# EUROPEAN PATENT APPLICATION

(11) **EP 4 748 436 A1**
(43) Date of publication of application: **27.05.2026**
(21) Application number: 25150930.3
(22) Date of filing: 09.01.2025
(51) Int. Cl.: A61N 1/36, G02C 7/04

(54) **CONTACT LENS**

(30) Priority: 20.11.2024 TW 113144551
(71) Applicant: Azurewave Technologies, Inc., 231 New Taipei City (TW)
(72) Inventor: LIAO, Yu-Hsuan, NEW TAIPEI CITY (TW); YEH, Tung-Lin, NEW TAIPEI CITY (TW); CHIEN, Huang-Chan, NEW TAIPEI CITY (TW)
(74) Representative: Viering, Jentschura & Partner mbB Patent- und Rechtsanwälte

(57) **Abstract**

A contact lens includes a glasses body (1) and an electronic component (2). The glasses body (1) can be worn on an eyeball (210), and includes a circular wearing portion (12) and an optical portion (11) surrounded by the circular wearing portion (12). The circular wearing portion (12) has a lower eyelid layout region (GA) in a C-shape. The electronic component (2) is disposed on the glasses body (1), and can move with the eyeball (210) through the glasses body (1). The electronic component (2) includes a stimulation unit (21) and a power supply unit (22) electrically coupled to the stimulation unit (21). The electrode module (211) is arranged in the lower eyelid layout region (GA), and can contact a body fluid around the eyeball (210). The boost control module (212) is electrically coupled to the electrode module (211), and can emit a pulse voltage signal through the electrode module (211). The pulse voltage signal is conducted by the body fluid to a lower eyelid (230) on a side of the eyeball (210).

## Description

### FIELD OF THE INVENTION

The present invention relates to a glasses, and more particularly to a contact lens.

### BACKGROUND OF THE INVENTION

In order to relieve eye discomfort caused by dry eye syndrome, a conventional contact lens uses electrodes to stimulate the sympathetic and parasympathetic nerves located in the orbit, so that the lacrimal glands located in the upper orbit can produce a tear response. In other words, the conventional contact lenses use tears to relieve dry eye syndrome. However, since current medical literature indicates that dry eye syndrome is not caused by insufficient tear secretion, the conventional contact lenses are not very effective in relieving dry eye syndrome.

### SUMMARY OF THE INVENTION

In response to the above-referenced technical inadequacy, the present invention provides a contact lens.

In order to solve the above-mentioned problems, one of the technical aspects adopted by the present invention is to provide a contact lens. The contact lens includes a glasses body and an electronic component. The glasses body is configured to be worn on an eyeball. The glasses body includes an optical portion and a circular wearing portion. The optical portion is surrounded by the circular wearing portion, and the circular wearing portion has a lower eyelid layout region in a C-shape. The electronic component is disposed on the glasses body. The electronic component is configured to move with the eyeball through the glasses body. The electronic component includes a stimulation unit and a power supply unit. The stimulation unit includes an electrode module and a boost control module. The electrode module is arranged in the lower eyelid layout region. The electrode module is configured to contact a body fluid around the eyeball. The boost control module is electrically coupled to the electrode module. The boost control module is configured to emit a pulse voltage signal through the electrode module, and the pulse voltage signal is conducted by the body fluid to a lower eyelid located on a side of the eyeball. The power supply unit is electrically coupled to the stimulation unit.

Therefore, in the contact lens provided by the present invention, by virtue of "the electrode module being arranged in the lower eyelid layout region and the electrode module being configured to contact a body fluid around the eyeball," and "the boost control module being configured to emit a pulse voltage signal through the electrode module, and the pulse voltage signal being conducted by the body fluid to a lower eyelid located on a side of the eyeball," the contact lens can effectively stimulate the secretion of oil from the meibomian glands to relieve dry eye syndrome.

These and other aspects of the present invention will become apparent from the following description of the embodiment taken in conjunction with the following drawings and their captions, although variations and modifications therein may be affected without departing from the spirit and scope of the novel concepts of the invention.

### BRIEF DESCRIPTION OF THE DRAWINGS

The described embodiments may be better understood by reference to the following description and the accompanying drawings, in which:
FIG. 1 is a schematic planar view of a contact lens according to the present invention;
FIG. 2 is a schematic planar view of an electronic component according to the present invention;
FIG. 3 is a schematic planar view of the contact lens according to the present invention worn on the eyes;
FIG. 4 is a schematic cross-sectional view taken along line IV-IV of FIG. 1;
FIG. 5 is a schematic enlarged view of part V of FIG. 4.
FIG. 6 is a schematic planar view of the contact lens according to the present invention in another implementation;
FIG. 7 is a schematic enlarged view of part VII of FIG. 6.
FIG. 8 is a schematic planar view of the contact lens according to the present invention in yet another implementation; and
FIG. 9 is a schematic planar view of the contact lens according to the present invention in still another implementation.

### DETAILED DESCRIPTION OF THE EXEMPLARY EMBODIMENTS

The present invention is more particularly described in the following examples that are intended as illustrative only since numerous modifications and variations therein will be apparent to those skilled in the art. Like numbers in the drawings indicate like components throughout the views. As used in the description herein and throughout the claims that follow, unless the context clearly dictates otherwise, the meaning of "a," "an" and "the" includes plural reference, and the meaning of "in" includes "in" and "on." Titles or subtitles can be used herein for the convenience of a reader, which shall have no influence on the scope of the present invention.

The terms used herein generally have their ordinary meanings in the art. In the case of conflict, the present document, including any definitions given herein, will prevail. The same thing can be expressed in more than one way. Alternative language and synonyms can be used for any term(s) discussed herein, and no special significance is to be placed upon whether a term is elaborated or discussed herein. A recital of one or more synonyms does not exclude the use of other synonyms. The use of examples anywhere in this specification including examples of any terms is illustrative only, and in no way limits the scope and meaning of the present invention or of any exemplified term. Likewise, the present invention is not limited to various embodiments given herein. Numbering terms such as "first," "second" or "third" can be used to describe various components, signals or the like, which are for distinguishing one component/signal from another one only, and are not intended to, nor should be construed to impose any substantive limitations on the components, signals or the like.

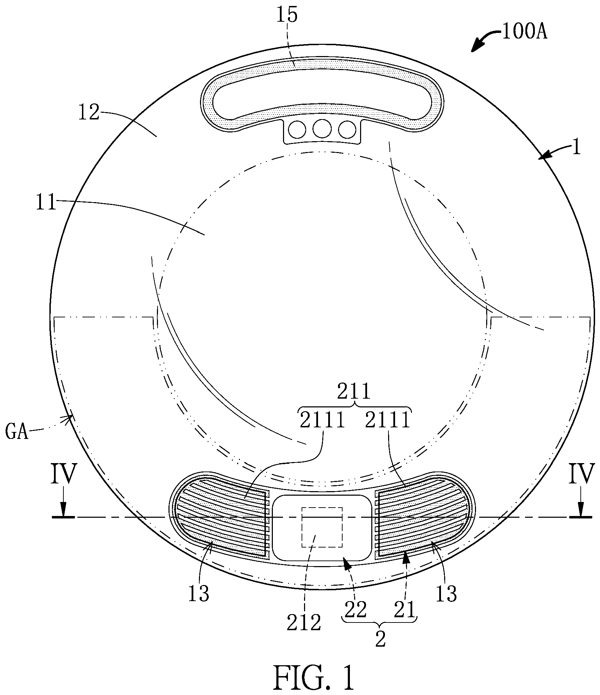

Referring to FIG. 1 to FIG. 9, the present invention provides a contact lens (e.g., 100A to 100C). Referring to FIG. 1 to FIG. 4, the embodiment provides a contact lens 100A (also referred to as an intelligent contact lens), and the contact lens 100A can be worn on an eye 200 of user (e.g., located between an eyeball 210, an upper eyelid 220, and a lower eyelid 230 of the eye 200) or implanted in the eye 200 (not shown in the figure) according to requirements.

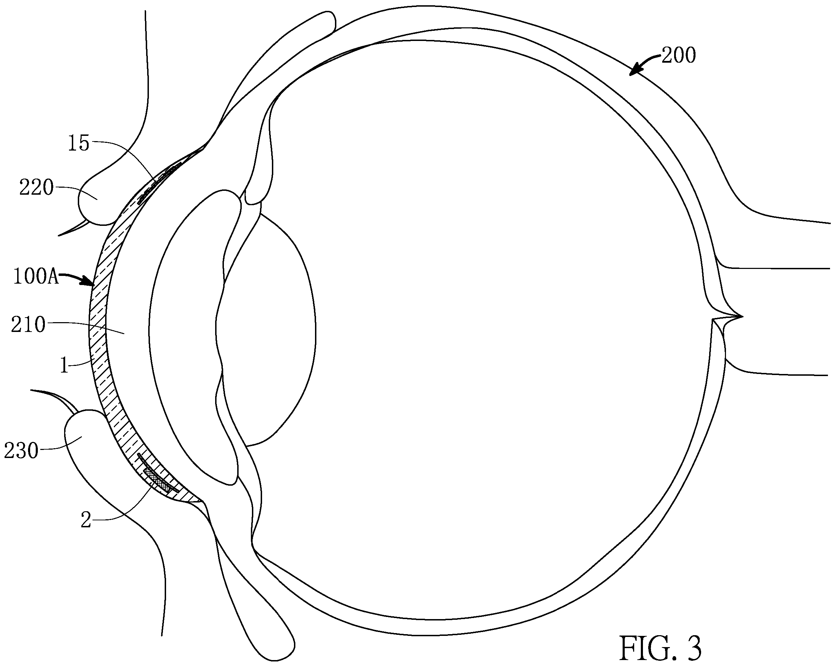

As shown in FIG. 3, the contact lens 100A in the present embodiment can have a function of correcting refractive errors, and the refractive errors include hyperopia, myopia, astigmatism, presbyopia, astigmatism, or presbyopia. Additionally, the contact lens 100A can be a cosmetic lens without a corrective function.

Referring to FIG. 1, FIG. 2, and FIG. 4, the contact lens 100A in the present embodiment includes a glasses body 1 and an electronic component 2 that is disposed on the glasses body 1. The following description describes the structure and connection relation of each component of the contact lens 100A.

The glasses body 1 in the present embodiment is formed through the curing of hydrogel or silicone hydrogel, with the aforementioned hydrogel being, for example, p-HEMA, but the present invention is not limited thereto. The glasses body 1 includes an optical portion 11 and a circular wearing portion 12, the optical portion 11 is surrounded by the circular wearing portion 12, and the optical portion 11 can be designed to either include or exclude a function of correcting refractive errors according to requirements.

It should be noted that, although the optical portion 11 in the present embodiment does not have any components embedded, the optical portion 11 can embed components according to requirements (e.g., when the contact lens 100A is applied in digital zoom devices), but the present invention is not limited thereto.

Furthermore, the optical portion 11 defines a central axis (not shown), and a center of the optical portion 11 and a center of the circular wearing portion 12 are both located on the central axis. The circular wearing portion 12 is connected to an outer edge of the optical portion 11 and is generally annular in shape, and the electronic component 2 is embedded inside the circular wearing portion 12. In addition, a production method of embedding the electronic component 2 in the circular wearing portion 12 (or a manufacturing method of the contact lens 100A) can be adjusted and changed according to requirements, and the present invention is not limited thereto.

In more detail, the circular wearing portion 12 has a lower eyelid layout region GA in a C-shape. The position of the lower eyelid layout region GA can be understood as corresponding to the location of the lower eyelid 230 of the eye 200, and the electronic component 2 is embedded within the lower eyelid layout region GA. When the contact lens 100A is worn on the eye 200, the lower eyelid layout region GA and the electronic component 2 correspond in position to a less sensitive region of the lower eyelid 230 of the eye 200, so as to effectively reduce the user's sensation of foreign objects.

In practice, the electronic component 2 is positioned between the eyeball of the eye 200 and the lower eyelid 230, and the electronic component 2 can move (or rotate) with the eyeball 210 through the glasses body 1. The electronic component 2 includes a stimulation unit 21 and a power supply unit 22 that is electrically coupled to the stimulation unit 21. The power supply unit 22 can provide power to the stimulation unit 21, and the stimulation unit 21 can use body fluid as a medium to emit a pulse voltage signal, so that a meibomian glands located at the lower eyelid 230 can be stimulated by the pulse voltage signal to produce lipids and proteins. Accordingly, the electronic component 2 can relieve the discomfort caused by dry eye syndrome through lipids and proteins.

More specifically, the stimulation unit 21 includes an electrode module 211 and a boost control module 212 that is electrically coupled to the electrode module 211. The electrode module 211 is disposed in the lower eyelid layout region GA, and the electrode module 211 can be used to contact a body fluid (e.g., tears or grease) around the eyeball 210.

In practice, an optimal position for the electrode module 211 is arranged with the Chengqi acupoint as the center. That is to say, the electrode module 211 is located directly below the pupil of the eyeball 210 when viewed from the front, and is situated between the eyeball 210 and the lower edge of the orbital bone, approximately 2.3 cm below the pupil, but the electrode module 211 is not limited thereto. In addition, the boost control module 212 can emit a pulse voltage signal through the electrode module 211, and the pulse voltage signal is used to be conducted by the body fluid to the lower eyelid 230 on one side of the eyeball 210.

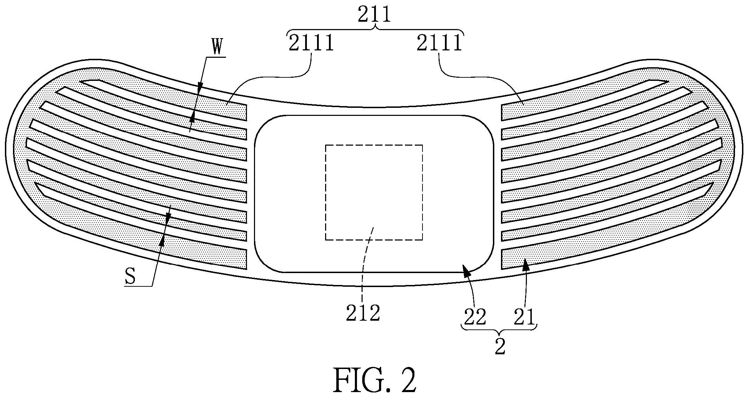

In an implementation, as shown in FIG. 2, the electrode module 211 may include two fingertip electrodes 2111, and the two fingertip electrodes 2111 are respectively arranged on two sides of the boost control module 212, so that the two fingertip electrodes 2111 can be symmetrically arranged with the boost control module 212.

In practice, each of the two fingertip electrodes 2111 has a plurality of conductors arranged in a comb shape, a width W of each of the conductors 2111 is within a range from 1 micron to 20 microns, and a gap S between two adjacent ones of the conductors 2111 is within a range from 10 nanometers to 100 micron, but the present invention is not limited thereto.

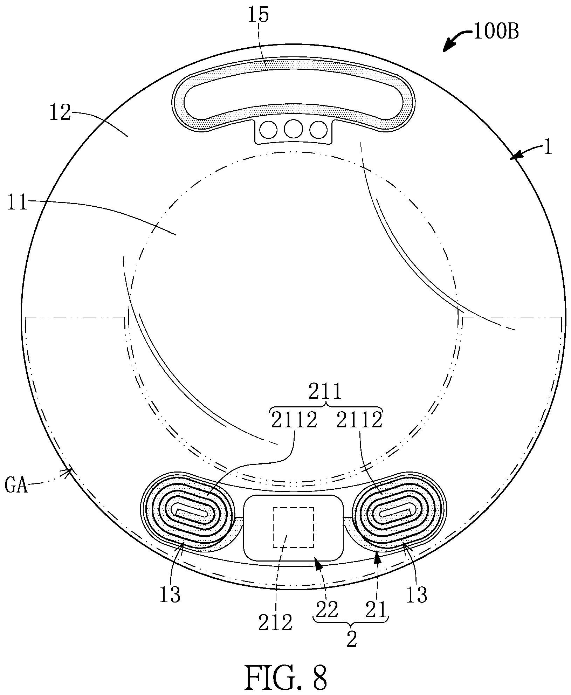

For example, in another implementation, as shown in FIG. 8, the electrode module 211 may include two coils 2112, the two coils 2112 are wound in a circular manner, and the two coils 2112 are respectively arranged on the two sides of the boost control module 212.

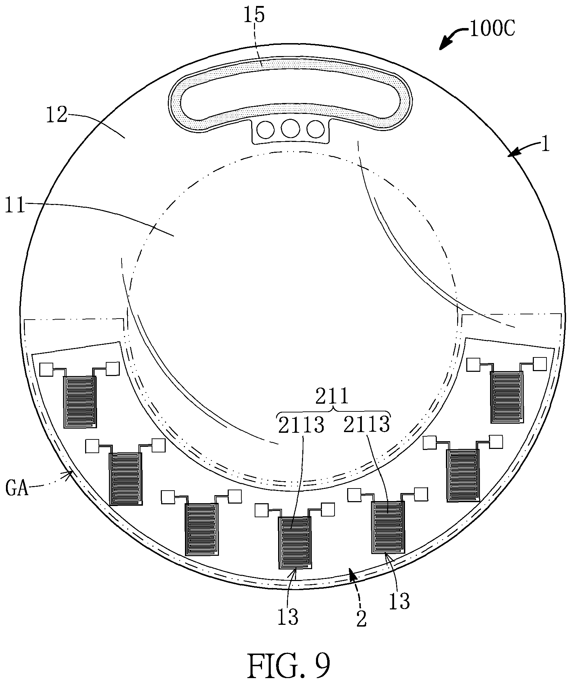

For another example, as shown in FIG. 9, the electrode module 211 may also include a plurality of electrode pads 2113 spaced apart from each other, and each of the electrode pads 2113 is in a rectangular shape and the electrode pads 2113 are configured in an array. The boost control module 212 can emit the pulse voltage signal through each of the electrode pads 2113. That is, the electrode pads 2113 can respectively emit the pulse voltage signals. For example, three electrode pads 2113 can sequentially emit the pulse voltage signals in turn.

In practice, to evenly distribute the electrode pads 2113, the electrode pads 2113 can embedded on a side of the circular wearing portion 12 facing the lower eyelid 230, and the boost control module 212 is embedded on a side of the circular wearing portion 12 facing the eyeball 210. Naturally, the positions of the electrode pads 2113 and the position of the boost control module 212 can also be interchanged depending on the situation.

Additionally, it should be specifically noted that, in practice, the boost control module 212 boosts a basic voltage provided by the power supply unit 22, so that the electrode module 211 to generate the pulse voltage signal.

Preferably, the basic voltage can be within a range from 1 volt to 5 volts, and the boost control module 212 can boost the basic voltage by 2 to 100 times to form the pulse voltage signal, but the present invention is not limited thereto.

The boost control module 212, in one implementation, can be a voltage doubler circuit, and the boost control module 212 can control a frequency of the pulse voltage signal to be adjusted within a range from 1 Hz to 1 MHz, but the present invention is not limited thereto. For example, the boost control module 212 can also be other circuits with the same effect.

Furthermore, it is worth noting that the pulse voltage signal is transmitted in conjunction with the body fluid and the electrode module 211 of the electronic component 2. However, to ensure the wearing comfort of the contact lens 100A, the glasses body 1 can completely encapsulate the electronic component 2.

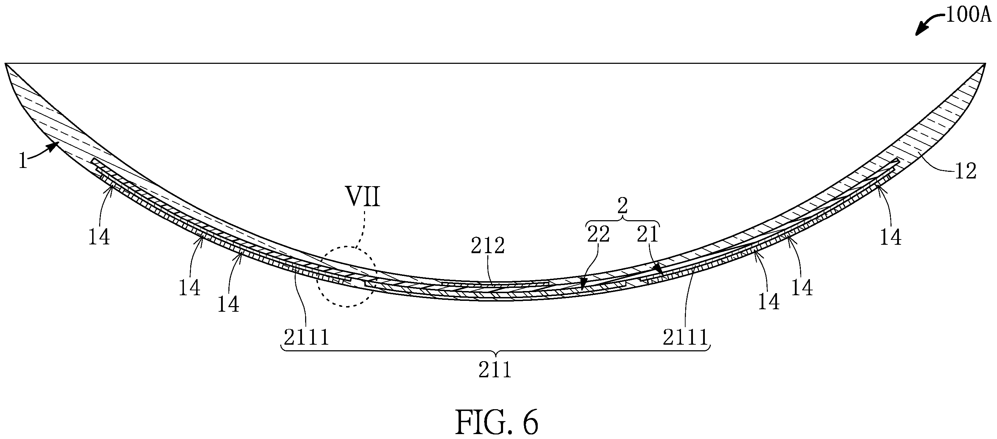

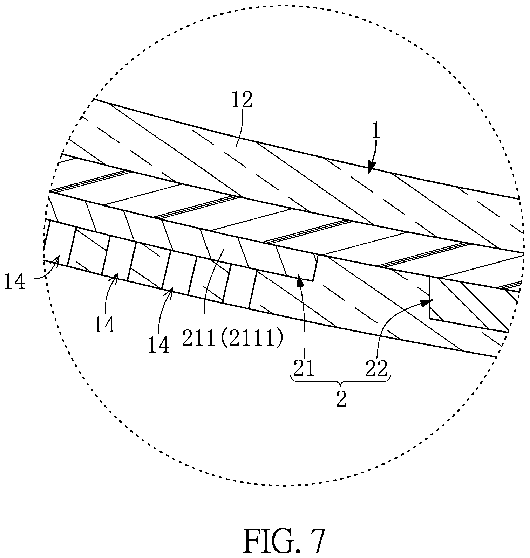

Specifically, as shown in FIG. 6 and FIG. 7, the glasses body 1 may further include a plurality of micro-pore structures 14. The positions of the micro-pore structures 14 correspond to locations within the lower eyelid layout region GA, and the micro-pore structures 14 penetrate through the circular wearing portion 12, so that the body fluid can pass through the micro-pore structures 14 and contact the electrode module 221, but the present invention is not limited thereto.

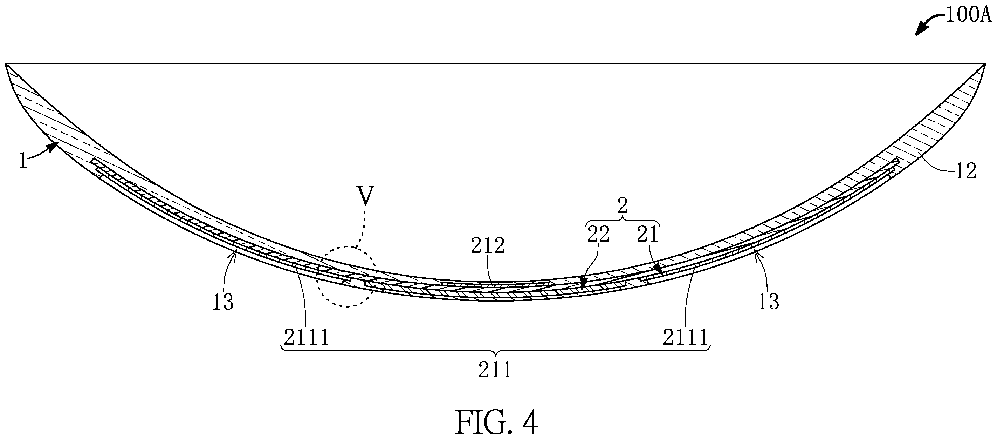

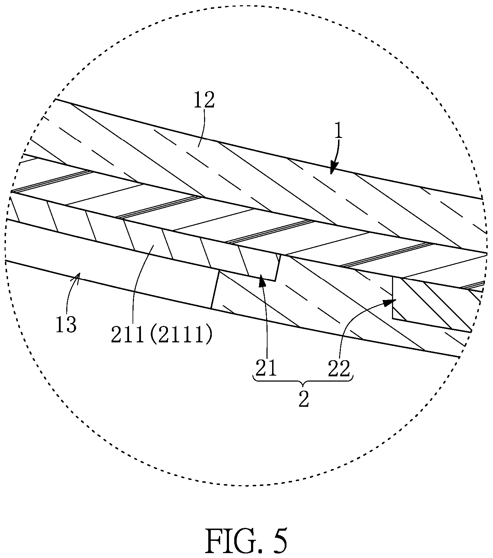

For example, as shown in FIG. 4 and FIG. 5, the glasses body 1 can also partially encapsulate the electronic component 2. Specifically, the glasses body 1 further includes a hollow portion 13. The hollow portion 13 is configured within the lower eyelid layout region GA of the circular wearing portion 12. The electrode module 211 can be exposed outside the glasses body 1 through the hollow portion 13 and contact the body fluid.

Moreover, according to the requirements, the contact lens 100A may also include an antenna 15 disposed on the circular wearing portion 12. The antenna 15 can be electrically coupled to the boost control module 212, and the antenna 15 can be used to receive and transmit a signal to control (for example, adjust) the boost control module 212, or to transmit information data from the boost control module 212.

Finally, it should be additionally noted that wearable devices for the eye area can be broadly categorized into two types: "direct contact with the eyeball" and "non-contact with the eyeball". In terms of common devices, frame glasses and eye masks belong to the "non-contact with the eyeball" category, while contact lenses belong to the "direct contact with the eyeball" category. The devices in these two categories have significant differences in functionality and configuration positions, resulting in completely different designs and layout configurations for the electronic components in each category. Furthermore, the electronic components between these two categories cannot be easily interchanged or repurposed.

More specifically, the electronic components in the direct contact with the eyeball category are necessarily positioned between the upper and lower eyelids and the eyeball, causing the electronic components to be in close proximity to or in direct contact with the eyeball. Any stimulating signals from these electronic components originate from the inner side of the eye. In contrast, the electronic components in the non-contact with the eyeball category indirectly contact the eyeball using the eyelid as a medium. In other words, any stimulating signals from these electronic components originate from the outer side of the eye.

Furthermore, within the direct contact with the eyeball category, the electronic components for the meibomian glands and those for the lacrimal glands are distinctly different. More specifically, the meibomian glands are located on the "inner side" of the eyelids, adjacent to the edge of the eyeball. Due to the unique position of the meibomian glands, the design of electronic components targeting them requires special attention to electrode placement and precision. The electrodes must be soft and able to comfortably adhere to the eyelid surface while avoiding pressure on the eyeball. Moreover, to effectively stimulate the meibomian glands, the device needs precise positioning capabilities to ensure accurate current delivery to the meibomian glands without unnecessarily affecting surrounding tissues. These design considerations make safety and comfort paramount for meibomian gland electrostimulation devices.

In contrast, the lacrimal glands are located in the upper outer side of the eye socket, positioned deeper and further from the eyeball compared to the meibomian glands. This requires lacrimal gland electrostimulation devices to be designed to cover a larger area around the eye socket or focus on specific nerve pathways to effectively stimulate the nerves related to the lacrimal glands. Additionally, since the stimulation target is at a deeper level, the frequency and intensity of electrical stimulation may need adjustment to ensure the stimulation penetrates and reaches the lacrimal glands. The design requirements make lacrimal gland electrostimulation devices more focused on structural stability and stimulation effectiveness.

In other words, any electronic component that is not positioned between the eyeball and eyelid and capable of stimulating the meibomian glands through pulse voltage (e.g., electronic components of frame glasses or eye masks) is not the electronic component referred to in the present invention.

### [Beneficial Effects of the Embodiment]

In conclusion, in the contact lens provided by the present invention, by virtue of "the electrode module being arranged in the lower eyelid layout region and the electrode module being configured to contact a body fluid around the eyeball," and "the boost control module being configured to emit a pulse voltage signal through the electrode module, and the pulse voltage signal being conducted by the body fluid to a lower eyelid located on a side of the eyeball," the contact lens can effectively stimulate the secretion of oil from the meibomian glands to relieve dry eye syndrome.

The foregoing description of the exemplary embodiments of the invention has been presented only for the purposes of illustration and description and is not intended to be exhaustive or to limit the invention to the precise forms disclosed. Many modifications and variations are possible in light of the above teaching.

The embodiments were chosen and described in order to explain the principles of the invention and their practical application so as to enable others skilled in the art to utilize the invention and various embodiments and with various modifications as are suited to the particular use contemplated. Alternative embodiments will become apparent to those skilled in the art to which the present invention pertains without departing from its spirit and scope.

## Claims

1. A contact lens, **characterized by** comprising:
a glasses body (1) configured to be worn on an eyeball (210), wherein the glasses body (1) includes an optical portion (11) and a circular wearing portion (12), the optical portion (11) is surrounded by the circular wearing portion (12), and the circular wearing portion (12) has a lower eyelid layout region (GA) in a C-shape; and
an electronic component (2) disposed on the glasses body (1), wherein the electronic component (2) is configured to move with the eyeball (210) through the glasses body (1), and the electronic component (2) includes:
a stimulation unit (21) including:
an electrode module (211) arranged in the lower eyelid layout region (GA), wherein the electrode module (211) is configured to contact a body fluid around the eyeball (210); and
a boost control module (212) electrically coupled to the electrode module (211), wherein the boost control module (212) is configured to emit a pulse voltage signal through the electrode module (211), and the pulse voltage signal is conducted by the body fluid to a lower eyelid (230) located on a side of the eyeball (210); and
a power supply unit (22) electrically coupled to the stimulation unit (21).

2. The contact lens according to claim 1, wherein the electrode module (211) includes two fingertip electrodes (2111), and the two fingertip electrodes (2111) are respectively arranged on two sides of the boost control module (212).

3. The contact lens according to claim 2, wherein each of the two fingertip electrodes (2111) has a plurality of conductors arranged in a comb shape, a width (W) of each of the conductors is within a range from 1 micron to 20 microns, and a gap (S) between two adjacent ones of the conductors is within a range from 10 nanometers to 100 micron.

4. The contact lens according to claim 1, wherein the boost control module (212) is a voltage doubler circuit, and the boost control module (212) is configured to adjust a frequency of the pulse voltage signal to adjust within a range from 1Hz to 1MHz.

5. The contact lens according to claim 1, wherein the power supply unit (22) is configured to provide a basic voltage, and the boost control module (212) is configured to increase the basic voltage by a factor within a range from 2 times to 100 times, so as to form the pulse voltage signal.

6. The contact lens according to claim 5, wherein the basic voltage is within a range from 1 volt to 5 volts.

7. The contact lens according to claim 1, wherein the electrode module (211) includes two coils (2112), and the two coils (2112) are respectively arranged on two sides of the boost control module (212).

8. The contact lens according to claim 1, wherein the electrode module (211) includes a plurality of electrode pads (2113) spaced apart from each other, and the boost control module (212) is configured to separately emit the pulse voltage signal through the electrode pads (2113).

9. The contact lens according to claim 1, wherein the electronic component (2) is covered by the glasses body (1), and the glasses body (1) includes a plurality of micro-pore structures (14); wherein the micro-pore structures (14) correspond in position to the lower eyelid layout region (GA), and the micro-pore structures (14) penetrate the circular wearing portion (12), so that the body fluid is configured to pass through the micro-pore structures (14) and contact the electrode module (211).

10. The contact lens according to claim 1, wherein the electronic component (2) is covered by the glasses body (1), and the glasses body (1) includes a hollow portion (13); wherein the hollow portion (13) is disposed in the lower eyelid layout region (GA) of the circular wearing portion (12), and the electrode module (211) is configured to be exposed to an outside of the glasses body (1) through the hollow portion (13) and contact the body fluid.
